# EUROPÄISCHE PATENTANMELDUNG

(11) **EP 1 745 755 A1**
(43) Veröffentlichungstag der Anmeldung: **24.01.2007**
(21) Anmeldenummer: 05026046.2
(22) Anmeldetag: 30.11.2005
(51) Int. Cl.: A61B 18/20, A61B 17/00

(54) **Verfahren zum Entfernen von Tätowierungen und Narben aus der Haut**

(30) Priorität: 18.07.2005 DE 102005034242
(71) Anmelder: Koziol, Karin-Sophie, 28209 Bremen (DE)
(72) Erfinder: Koziol, Karin-Sophie, 28209 Bremen (DE)

(57) **Zusammenfassung**

Die Erfindung betrifft ein Verfahren zur Entfernen von Tätowierungen und Narben, bestehend aus zwei Schritten. Ein erster Schritt ist das Öffnen der Oberhaut mittels einer Fräseinrichtung. Die Öffnungen sind einzeln, punktuell und nahezu kreisförmig. Die Abstände zwischen den einzelnen Öffnungen betragen mindestens den 1,5 fachen Durchmesser der Öffnungen. Die Tiefe beschränkt sich auf die Oberhaut. Die Fräseinrichtung benetzt die Öffnungen mit einer Flüssigkeit und ein Gas aus einem Luft-Sauerstoffgemisch fördert die Heilung der Wunde. Nach der Wundheilung bricht das Laserlicht in einem zweiten Schritt die noch in der Oberfläche der Haut zurückgebliebenen Farbpigmente auf. Das Gewebe stößt diese kürzeren Molekülketten ab und die Lymphkanäle führen diese ab.

## Beschreibung

Die Erfindung betrifft ein Verfahren zum Entfernen von Tätowierungen und Narben aus der Haut durch das Herbeiführen einer natürlichen Hautreaktion, die eingeleitet wird durch die Erzeugung von nahezu kreisförmigen Öffnungen der Haut mittels einer Fräseinrichtung, verbunden mit einer Bestrahlung der gleichen Hautabschnitte mit Laserlicht.

Die Herstellung von Tätowierungen beruht auf das Einbringen von Farbpigmenten in die Haut. Beim professionellen Tätowieren werden diese Farbpigmente in der Regel in einer Tiefe von ca. 1,5 mm gleichmäßig eingebracht. Die hierfür verwendeten Tätowiermaschinen mit Nadeln garantieren diese gleichmäßige Tiefe. Es entsteht durch die Farbpigmente eine optische Darstellung, die sich dauerhaft in der Haut befindet und durch die Hornhaut sichtbar ist. Bei nicht-professionell eingebrachten Tätowierungen befinden sich die Farbpigmente meist in unterschiedlichen Tiefen.

Bekannt sind Verfahren zum Entfernen von Tätowierungen.
In der Patentschrift mit der Nummer: 385666 ausgegeben vom Reichspatentamt am 26. November 1923 ist ein "Mittel zum Entfernen von künstlich unter die Haut gebrachten Farbstoffen (Tätowierungen)", bestehend aus drei Flüssigkeiten, beschrieben. Die beiden ersten Flüssigkeiten werden einige Male auf die Haut aufgetragen, "bis sich die erste Hautschicht, unter Zuhilfenahme eines messerartig zugeschnittenen Hölzchens, leicht abschälen lässt". Anschließend wird die dritte Flüssigkeit aufgebracht und die Wunde mit einem Pflaster luftdicht abgeschlossen. Mit der Abnahme des Pflasters reißt der Teil der Haut ab, in der sich Farbstoffe befinden. Die Anwendung dieses in der Patentschrift beschriebenen Mittel, enthält eine große Gefahr für die Gesundheit, denn die Hautteile, die Farbstoffe beinhalten, werden regelrecht von der Flüssigkeit angegriffen und vom Pflaster weggenommen.

In der Patentschrift DE 43 08 824 C1 ist ein Verfahren zum Entfernen von Tätowierungen unter Anwendung von Wärme beschrieben. Mit einer Elektrode wird in die Tätowierung ein hochfrequenter Strom zur Erwärmung des Gewebes eingebracht. An der Einstichöffnung gerinnt die Zellsubstanz durch die Wärme und nachdem die Schorfschicht abgefallen ist, entfernt man die Narbe durch Abschleifen.

Das Gebrauchsmuster DE 202 09 676 U1 beschreibt das Auftragen von pflanzlichen Extrakten, sowie von Kochsalz zum Entfernen von Tätowierungen. Die Haut entzündet sich, wodurch die Farbpartikel abgestoßen werden.

Die Offenlegungsschrift DE 100 56 114 A1 beschreibt ein Verfahren, das mit einer Nadel oder mit mehreren Nadeln die Farbpigmente in der Haut mechanisch zerstört. In der Beschreibung dieser Offenlegungsschrift sind die unterschiedlichen Methoden zur Entfernung einer Tätowierung beschrieben.
Hier sind erwähnt:
- das "Cover-Up" Verfahren, das alte in ein neues Bild einbeziehen
- das Absterbenlassen der gestochenen Haut durch das Auftragen eines Fliegendüsensekrets
- das Ausschneiden von Tätowierungen aus der Haut
- das Abhobeln oder Abschleifen der Haut
- die Zerstörung der Farbpigmente mittels Laserlicht
- das Waterjet-Cutting: die Haut über der Tätowierung wird angeschnitten und angehoben, um die darunter liegenden Farbpigmente wegzuspülen.

Die Patentschrift DE 696 06 176 T2 beschreibt eine Vorrichtung zur Mikroabtragung von Hautgewebe durch einen Partikelstrahl. Die Partikel aus Korund befinden sich in einem Wegwerfbehälter und werden nach dem Abtragen in einem identischen Behälter wieder aufgenommen.

Bisher bekannte Verfahren zum Entfernen von Tätowierungen sind in der Regel verbunden mit relativ starken Eingriffen in die Haut bis hin zu tieferen Schichten. Es entsteht dadurch die Möglichkeit der Narbenbildung, da sich die Farbpigmente doch zum Teil tief, möglicherweise bis in der Fettschicht befinden.

Die Aufgabe der vorliegenden Erfindung ist es nun, durch einen möglichst schonenden Eingriff in die Haut eine natürliche Reaktion hervor zurufen, in der Gestalt, dass die Haut die Farbpigmente abstößt, bzw. absorbiert.

Das vorliegende Verfahren umfasst zwei Schritte, wobei der erste Schritt die Oberschicht der Haut angreift und dadurch die natürliche Reaktion der Haut zur Selbstregeneration verbunden mit einer abstoßenden Wirkung der Farbpigmente auslöst. Ein zweiter Schritt, der mittels Laserlicht, das die nach der Selbstregeneration noch in der Oberfläche der Haut befindlichen Farbpigmente aufbricht, wodurch diese, vom Gewebe abgestoßen, über die Lymphkanäle abgeführt werden können.

Auch ist das Verfahren in der gleichen Weise geeignet, kelloide Narben, die sich in der Oberhaut der Haut befinden, zu entfernen.

Der Eingriff in die Haut erfolgt durch eine Fräseinrichtung mit einem Fräser zur Erzeugung von Öffnungen. Der Fräser besitzt eine plane Fläche zum Fräsen der Öffnungen. Es entstehen dadurch nahezu kreisförmig ausgebildete Öffnungen. Es ist vorzugsweise ein Fräser zu verwenden, der Öffnungen mit einem Durchmesser von ca. 3 mm hervorbringt. Die einzelnen Öffnungen dürfen nicht zu nah beieinander liegen. Die Abstände der Öffnungen sollen mindestens den 1,5 fachen Durchmesser der einzelnen Öffnungen betragen. Auf diese Weise findet keine flächige Hautabtragung statt.

Die Tiefe der Öffnungen begrenzt sich auf das Öffnen der Oberhaut und beträgt ca. bis 0,05 mm. Die Oberhaut (Epidermis) kann sich dadurch narbenfrei regenerieren. Beträgt die Tiefe der weggenommenen Haut mehr als 0,05 mm, dauert der Regenerationsprozess entsprechend länger. Außerdem ist das Schmerzempfinden bei der Behandlung größer.

Während des Fräsvorgangs wird gleichzeitig mit dem Fräser die Oberfläche der Öffnung mit einer Flüssigkeit benetzt. Die in dieser Flüssigkeit enthaltene Milchsäure reizt die Haut, sie dringt in tiefere Schichten ein und verursacht eine Verzögerung der Wundheilung. Die Öffnungen bleiben länger geöffnet. Die Milchsäure verhindert ebenfalls die Entstehung von bakteriellen Taschen in den Öffnungen. Die Chancen der Entwicklung von Entzündungen werden vermindert.

Es hat sich auch als vorteilhaft herausgestellt, während oder zeitnah zu dem Fräsvorgang die Öffnungen mit einem Luft-Sauerstoffgemisch zu beaufschlagen. Die Wirkung von Sauerstoff verhindert ebenfalls den Einschluss von Bakterien in den Öffnungen. Erfahrungen im medizinischen Bereich haben ergeben, das durch die Zufuhr von Sauerstoff Wunden schneller heilen und der Bildung von Narben entgegengewirkt wird.

Der oben beschriebene erste Schritt löst eine Reaktion aus, die Farbpigmente zur Oberfläche hin wandern lässt. Ein Teil der Farbpigmente kann auf diese Weise von der Oberfläche entfernt werden, ein Teil verbleibt in der Haut, da durch die Wundheilung die abstoßende Reaktion gebremst bzw. gestoppt wird. Die Farbpigmente, die sich noch unter der Hautoberfläche befinden, werden in einem zweiten Schritt mit Laserlicht bestrahlt. Dieses energiereiche Laserlicht spaltet diese Farbpigmente auf, kürzere Molekülketten entstehen, die vom Gewebe abgestoßen und über die Lymphkanäle abgeführt werden.

Dieses Verfahren ist ebenfalls geeignet, Narben, die sich bis zu einer Tiefe von 0,05 mm in der Oberhaut befinden, zu entfernen. Die Haut führt den Regenerationsprozess durch, wobei neue Zellen entstehen. Sowohl die Flüssigkeit als auch das Schutzgas fördern den Ablauf. Die Intensität des Laserlichtes ist zu reduzieren, um die Heilung zu stützen.

Das in den Patentansprüchen beschriebene Verfahren hat den Vorteil eines minimalen Eingriffes in die Haut. Die bei dem Eingriff eingebrachte benetzende Flüssigkeit in Kombination mit dem Luft-Sauerstoffgemisch erzeugt eine optimale Abstoßreaktion und kontrollierte Wundheilung. Die in der Haut, auch in unterschiedlichen Tiefen, befindlichen Farbpigmente werden zur Oberfläche hin abgestoßen. Ein weiterer Vorteil ist das nur punktuelle Öffnen der Haut bei einer geringen Tiefe der Öffnungen. Nach der Wundheilung bricht das Laserlicht die noch in der Oberfläche der Haut zurückgebliebenen Farbpigmente auf. Das Gewebe stößt die kürzeren Molekülketten ab und die Lymphkanäle führen diese ab.

## Patentansprüche

1. Verfahren zum Entfernen von Tätowierungen und Narben aus der Haut, **dadurch gekennzeichnet, dass** dieses Verfahren aus zwei Schritten besteht, die nach einander zur Ausführung kommen. Ein erster Schritt beinhaltet das Öffnen der Haut, ein zweiter Schritt die Bestrahlung der gleichen Hautstelle mit Laserlicht.
Der erste Schritt, das Öffnen der Haut hat folgende Merkmale:
a. die Öffnungen im Bereich der Oberhaut erzeugt eine Fräseinrichtung
b. die Öffnungen erhalten einen Durchmesser von der Größe der Fräsfläche des Fräsers
c. die Öffnungen sind nahezu kreisförmig
d. die Abstände zwischen den Öffnungen betragen mindestens den 1,5 fachen Durchmesser der einzelnen Öffnungen
e. die Tiefe der Öffnungen beschränkt sich auf die Oberhaut
f. mit der Fräseinrichtung wird während des Fräsens eine Flüssigkeit in die Öffnungen gebracht
g. die Erzeugung der Öffnungen geschieht zeitnah unter Einwirkung eines Schutzgases.
Der zweite Schritt beinhaltet das Bestrahlen der gleichen Hautteile:
h. mit einem Laser.
i. Die Bestrahlung mit dem Laserlicht erfolgt während und nach der Heilungsperiode der Haut.

2. Verfahren nach Anspruch 1, **dadurch gekennzeichnet, dass** die Fräseinrichtung mit einem Fräser ausgestattet ist, der eine plane Fräsfläche von mindestens 3 mm Durchmesser aufweist.

3. Verfahren nach Anspruch 1, **dadurch gekennzeichnet, dass** die Tiefe der Öffnungen sich im wesentlichen auf 0,05 mm in der Oberhaut (Epidermis) beschränken.

4. Verfahren nach Anspruch 1, **dadurch gekennzeichnet, dass** mit der Fräseinrichtung eine Flüssigkeit in die Öffnungen gebracht wird, die desinfizierend wirkt und die Öffnungen länger geöffnet hält.

5. Verfahren nach Anspruch 1, **dadurch gekennzeichnet, dass** als Schutzgas ein Luftgemisch angereichert mit Sauerstoff Verwendung findet, das Bakterien fernhält und die Wundheilung unterstützt.

6. Verfahren nach Anspruch 1, **dadurch gekennzeichnet, dass** es kelloide Narben entfernt.
